# FASCICULE DE BREVET EUROPEEN

(11) **EP 3 443 070 B1**
(45) Date de publication et mention de la délivrance du brevet: **15.09.2021**
(21) Numéro de dépôt: 17719866.0
(22) Date de dépôt: 10.04.2017
(51) Int. Cl.: C12N 1/12, C12M 1/00, B01D 53/62

(54) **PROCÉDÉ DE CULTURE DE MICRO-ALGUES ET/OU DE CYANOBACTÉRIES À PARTIR D'EFFLUENTS DE GAZ INDUSTRIELS CONTENANT DU DIOXYDE DE CARBONE ET INSTALLATION POUR LA MISE EN OEUVRE DE CE PROCÉDÉ**
VERFAHREN ZUR KULTIVIERUNG VON MIKROALGEN BZW. ZYANOBAKTERIEN UNTER VERWENDUNG VON INDUSTRIELLEN CO2 ENTHALTENDEN GASABLEITUNGEN UND INSTALLATION ZUR DURCHFÜHRUNG DES VERFAHRENS
METHOD FOR CULTURING MICROALGAE AND/OR CYANOBACTERIA FROM INDUSTRIAL GAS EFFLUENTS CONTAINING CARBON DIOXIDE AND FACILITY FOR IMPLEMENTING SAID METHOD

(30) Priorité: 12.04.2016 FR 1653200
(43) Date de publication de la demande: 20.02.2019
(73) Titulaire: ITALCEMENTI S.p.A., 24126 Bergamo (IT); Algosource Technologies, 44600 Saint-Nazaire (FR)
(72) Inventeur: CORAZZA, Fabio, 24020 Gorle (IT); LOMBARD, Christophe, 61000 Alençon (FR); LEURANGUER, Jérôme, 44600 Saint-Nazaire (FR); LEBORGNE, François, 56300 Pontivy (FR); LEPINE, Olivier, 44400 Reze (FR)
(74) Mandataire: De Gregori, Antonella
(86) Numéro de dépôt international: PCT/FR2017/050852
(87) Numéro de publication internationale: WO 2017/178743

(56) Documents cités:
- WO-A2-2008/107896
- WO-A2-2008/107896
- US-A1- 2008 220 486
- CLEMENS G BORKENSTEIN ET AL: "Cultivation ofwith flue gas derived from a cement plant", JOURNAL OF APPLIED PHYCOLOGY, KLUWER ACADEMIC PUBLISHERS, DO, vol. 23, no. 1, 9 juillet 2010 (2010-07-09), pages 131-135, XP019881958, ISSN: 1573-5176, DOI: 10.1007/S10811-010-9551-5
- SHAHRIAR RAHMAN ET AL: "Algae based biofuel production from CO 2 obtained from fixed chimney brickfields in Bangladesh", ELECTRICAL&COMPUTER ENGINEERING (ICECE), 2012 7TH INTERNATIONAL CONFERENCE ON, IEEE, 20 décembre 2012 (2012-12-20), pages 754-757, XP032338050, DOI: 10.1109/ICECE.2012.6471660 ISBN: 978-1-4673-1434-3
- JIRI DOUCHA ET AL: "Utilization of flue gas for cultivation ofmicroalgae (Chlorella sp.) in an outdoor open thin-layer photobioreacto", JOURNAL OF APPLIED PHYCOLOGY, KLUWER, DORDRECHT, NL, vol. 17, no. 5, 1 octobre 2005 (2005-10-01), pages 403-412, XP008159981, ISSN: 0921-8971, DOI: 10.1007/S10811-005-8701-7
- CHENG JUN ET AL: "Improving growth rate of microalgae in a 1191 m2 raceway pond to fix CO2 from flue gas in a coal-fired power plant", BIORESOURCE TECHNOLOGY, vol. 190, 1 août 2015 (2015-08-01), pages 235-241, XP029130274, ISSN: 0960-8524, DOI: 10.1016/J.BIORTECH.2015.04.085
- CUELLAR-BERMUDEZ SARA P ET AL: "Photosynthetic bioenergy utilizing CO2: an approach on flue gases utilization for third generation biofuels", JOURNAL OF CLEANER PRODUCTION, vol. 98, 1 juillet 2015 (2015-07-01), pages 53-65, XP029181936, ISSN: 0959-6526, DOI: 10.1016/J.JCLEPRO.2014.03.034
- CLEMENS G BORKENSTEIN ET AL: "Cultivation of Chlorella emersonii with flue gas derived from a cement plant", JOURNAL OF APPLIED PHYCOLOGY, KLUWER ACADEMIC PUBLISHERS, DO, vol. 23, no. 1, 9 juillet 2010 (2010-07-09), pages 131-135, XP019881958, ISSN: 1573-5176, DOI: 10.1007/S10811-010-9551-5 cité dans la demande
- M. OLOFSSON ET AL: "Baltic Sea microalgae transform cement flue gas into valuable biomass", ALGAL RESEARCH, vol. 11, 1 septembre 2015 (2015-09-01), pages 227-233, XP055380224, NL ISSN: 2211-9264, DOI: 10.1016/j.algal.2015.07.001
- TALEC AMÉLIE ET AL: "Effect of gaseous cement industry effluents on four species of microalgae", BIORESOURCE TECHNOLOGY, vol. 143, 30 mai 2013 (2013-05-30), pages 353-359, XP028680198, ISSN: 0960-8524, DOI: 10.1016/J.BIORTECH.2013.05.104
- Indra Suryata ET AL: "GEOTHERMAL CO2 BIO-MITIGATION TECHNIQUES BY UTILIZING MICROALGAE AT THE BLUE LAGOON, ICELAND", PROCEEDINGS, 1 février 2010 (2010-02-01), XP055323442, Extrait de l'Internet: URL:http://www.bluelagoon.com/files/resear ch-studies/blue-lagoon-research/geothermal -co2-bio-mitigation-techniques-by-utilizin g-microalgae-at-the-blue-lagoon-iceland.pd f [extrait le 2016-11-28]
- CHEAH WAI YAN ET AL: "Biosequestration of atmospheric CO2 and flue gas-containing CO2 by microalgae", BIORESOURCE TECHNOLOGY, vol. 184, 1 mai 2015 (2015-05-01), pages 190-201, XP029205301, ISSN: 0960-8524, DOI: 10.1016/J.BIORTECH.2014.11.026
- CLEMENS G BORKENSTEIN ET AL: "Cultivation ofwith flue gas derived from a cement plant", JOURNAL OF APPLIED PHYCOLOGY, KLUWER ACADEMIC PUBLISHERS, DO, vol. 23, no. 1, 9 July 2010 (2010-07-09), pages 131-135, XP019881958, ISSN: 1573-5176, DOI: 10.1007/S10811-010-9551-5
- SHAHRIAR RAHMAN ET AL: "Algae based biofuel production from CO 2 obtained from fixed chimney brickfields in Bangladesh", ELECTRICAL&COMPUTER ENGINEERING (ICECE), 2012 7TH INTERNATIONAL CONFERENCE ON, IEEE, 20 December 2012 (2012-12-20), pages 754-757, XP032338050, DOI: 10.1109/ICECE.2012.6471660 ISBN: 978-1-4673-1434-3
- JIRI DOUCHA ET AL: "Utilization of flue gas for cultivation ofmicroalgae (Chlorella sp.) in an outdoor open thin-layer photobioreacto", JOURNAL OF APPLIED PHYCOLOGY, KLUWER, DORDRECHT, NL, vol. 17, no. 5, 1 October 2005 (2005-10-01), pages 403-412, XP008159981, ISSN: 0921-8971, DOI: 10.1007/S10811-005-8701-7

## Description

### DOMAINE DE L'INVENTION

La présente invention concerne le domaine des procédés de culture des micro-algues, plus particulièrement un procédé de culture de micro-algues dans un photobioréacteur.

### ETAT DE LA TECHNIQUE ANTERIEURE

Pour croître et se développer, la biomasse, telle que les micro-algues, a besoin d'éléments nutritifs minéraux, mais aussi d'eau, de CO₂ et de lumière, pour réaliser sa photosynthèse.

Dans un contexte de lutte contre le réchauffement climatique et du fait d'une consommation spécifique de CO₂ plus importante au m² que les plantes terrestres (environ 10 fois plus importante), ont été conduites depuis une vingtaine d'années de nombreuses recherches visant à développer des procédés de captage et de valorisation de gaz et fumées d'origine industrielle riches en CO₂ via l'utilisation de microalgues afin de produire des molécules d'intérêt ou des biocarburants. Récemment, il a été proposé d'utiliser des effluents gazeux industriels tels que les effluents gazeux de cimenteries ou de centrales électriques à charbon (CHENG Jun et al., Bioressource Technology, 190, 2015, pp 235-241) pour enrichir les milieux de culture de certaines micro-algues en dioxyde de carbone.

Cependant, selon leur origine, les effluents gazeux industriels peuvent être chargés en composants inhibant la croissance des micro-organismes concernés, ou réduisant les capacités ultérieures d'utilisation de ces micro-organismes.

Par exemple, le document de C.G.Borkenstein (J. Appl. Phycol., 23, 2010, pp 131-135 qui décrit un procédé utilisant comme source carbonée de culture d'une algue verte (*Chlorella emersonii*) un effluent gazeux de cimenterie, conclut que le plomb s'accumule dans la biomasse algale (avec un taux trois fois supérieur à la valeur de la référence) qui ne doit donc pas être utilisée en tant que complément alimentaire ni nourriture pour les animaux.

### BUT DE L'INVENTION

Un premier but de l'invention est donc de proposer un procédé de culture de microalgues à partir d'effluent de gaz industriel contenant du dioxyde de carbone qui n'altère pas les propriétés de ces micro-algues et permette leur valorisation, notamment dans l'alimentation animale ou humaine.

Un autre but de l'invention est de proposer un procédé de culture de micro-algues à partir d'effluent de gaz industriel contenant du dioxyde de carbone qui soit applicable également à des cyanobactéries.

### DESCRIPTION DE L'INVENTION

A cet effet la présente invention concerne un procédé de culture de micro-algues et/ou de cyanobactéries dans un photobioréacteur renfermant un milieu nutritif incluant une source carbonée, la source carbonée est un effluent de gaz industriel contenant du dioxyde de carbone, et le procédé comprend les étapes successives suivantes :
a) - Le captage de l'effluent gazeux industriel (fumées) humide contenant du dioxyde de carbone,
b) - Le séchage/purification dudit effluent gazeux par refroidissement, provoquant la condensation de la fraction humide et l'obtention d'un effluent gazeux sec,
c) - La séparation du condensat de l'effluent gazeux sec contenant du dioxyde de carbone,
d) - le suivi du pH du milieu de culture dans le photobioréacteur renfermant une ou plusieurs souches de micro-algues et/ou cyanobactéries, avec détermination d'une gamme de pH optimale pour la croissance desdites souches,
e1) - L'enrichissement en dioxyde de carbone (carbonatation) d'une solution nutritive des micro-algues et/ou cyanobactéries au moyen dudit effluent sec/purifié, suivi, en fonction du pH du milieu de culture, de l'injection de ladite solution nutritive enrichie en dioxyde de carbone dans le photobioréacteur,
ou e2) - l'injection directe de l'effluent sec contenant le dioxyde de carbone dans le photobioréacteur,
f) - La récolte de la biomasse produite en vue de sa valorisation,
caractérisé en ce que l'effluent gazeux est un effluent issu de l'industrie cimentière, plus particulièrement capté dans le conduit de la cheminée d'une cimenterie, avant son rejet dans l'atmosphère, en ce que le refroidissement de l'effluent est effectué à une température inférieure ou égale à 5°C, et en ce que les micro-algues ou cynanobactéries sont choisies parmi les souches suivantes : *Arthrospira platensis* (spiruline), *Chlorella vulgaris* ou *Nannochloropsis oculata.*

L'importance de l'étape de séchage de l'effluent gazeux, permettant l'obtention d'un gaz sec, réside également dans l'élimination de tous les éléments entrainés par condensation de la fraction humide du gaz, entrainant simultanément la réduction des poussières résiduelles et des éléments solubles dans la vapeur d'eau. Cette étape est ainsi également qualifiée d'étape de purification de l'effluent gazeux.

Avantageusement au moins une partie des calories récupérées lors du refroidissement de l'effluent brut sont dirigées vers le photobioréacteur et/ou vers le gaz sec, afin de remonter sa température.

De préférence, la teneur en vapeur d'eau du gaz sec sous pression atmosphérique est inférieure ou égale à 3 %, de préférence inférieure ou égale à 1 %.

Selon un premier mode de réalisation de l'invention, le gaz sec vient enrichir en CO₂ la solution nutritive avant son entrée dans le photobioréacteur.

Selon un second mode de réalisation de l'invention, le gaz sec est injecté directement dans le photobioréacteur, la solution nutritive étant introduite séparément.

L'effluent gazeux, étant un effluent issu de l'industrie cimentière, plus particulièrement capté dans le conduit de la cheminée d'une cimenterie, avant son rejet dans l'atmosphère, le procédé selon l'invention permet ainsi le recyclage des fumées de cimenterie, avec avantageusement une valorisation également de la chaleur fatale du procédé de clinkérisation.

Les exemples présentés plus loin montrent qu'un tel effluent, issu de cimenterie, n'est pas, contrairement à l'enseignement de l'état de la technique mentionné en introduction, préjudiciable à la valorisation ultérieure des micro-algues ainsi produites, même à une valorisation dans le domaine alimentaire.

Le fonctionnement d'une cimenterie étant continu, le procédé de culture selon l'invention peut être réalisé en continu.

Plus particulièrement, il a été observé, de manière surprenante, que les micro-algues et/ou cyanobactéries cultivées selon le procédé de l'invention, choisies parmi les souches suivantes : *Arthrospira platensis* (spiruline), *Chlorella vulgaris* ou encore *Nannochloropsis oculata,* sont résistantes et ne présentent pas d'accumulation en éléments métalliques, dont le plomb.

L'introduction du gaz sec susmentionné est asservie au pH du milieu de culture. En effet, une quantité trop importante de CO₂ acidifie le milieu. De préférence, le pH du milieu de culture est régulé entre 6 et 10, de préférence entre 7 et 8 pour la souche *Chlorella vulgaris,* de préférence entre 9 et 10 pour la souche *Arthrospira platensis.*

Il est apparu qu'au moins une fraction du condensat est introduite dans le photobioréacteur, sans que cela soit préjudiciable : ni en ce qui concerne le rendement de la culture, ni à la qualité des micro-algues ou cyanobactéries.

De préférence, le refroidissement de l'effluent est effectué à une température inférieure ou égale à 5 °C, de préférence à une température inférieure ou égale à 2 °C.

Après séparation d'avec le condensat, le gaz sec est avantageusement réchauffé avant bullage dans le milieu de culture, de préférence, à une température supérieure ou égale à 20 °C et inférieure ou égale à la température optimale (*Arthrospira platensis* (spiruline) ≤ à 35 °C ; *Chlorella vulgaris ≤* 25 °C) de croissance des microalgues et/ou cyanobactéries ainsi cultivées.

De manière avantageuse, les différentes étapes comprenant le séchage/purification de l'effluent par refroidissement, la séparation de l'effluent sec et du condensat, l'enrichissement en dioxyde de carbone (carbonatation) du milieu de culture des micro-algues et l'injection dudit milieu de culture enrichi en dioxyde de carbone dans un photobioréacteur sont réalisées sous une pression relative comprise entre 0,1 et 2 bar, de préférence sous une pression relative comprise entre 0,2 et 1 bar.

Le procédé selon la présente invention peut être mis en œuvre dans une installation qui comprend les éléments suivants, de l'amont vers l'aval :
- des moyens de prélèvement, tel qu'une pompe de prélèvement, d'effluent brut industriel ;
- un séparateur gaz/liquide comprenant un échangeur de chaleur comportant un module de refroidissement de l'effluent brut ;
- ledit séparateur gaz/liquide étant connecté d'une part, pour la fraction liquide, à un organe de prélèvement du condensat, et d'autre part, pour la fraction gazeuse, à des moyens d'introduction de l'effluent sec/purifié directement dans le photobioréacteur ou dans un contacteur gaz /liquide renfermant une solution nutritive des micro-algues et/ou cyanobactéries destiné à enrichir en dioxyde de carbone ladite solution nutritive ;
- des moyens d'injection, telle qu'une pompe doseuse ou telle qu'une pompe péristaltique, du milieu de culture enrichi en dioxyde de carbone dans un photobioréacteur,
- un photobioréacteur contenant un milieu de culture renfermant une ou plusieurs souches de micro-algues et/ou cyanobactéries, équipé d'un pH-mètre de mesure dudit milieu de culture, et comprenant un module d'alimentation en solution nutritive de la biomasse et un module de prélèvement de la biomasse produite dans ledit photobioréacteur.

De préférence, les organes de prélèvement de l'effluent brut gazeux industriel sont aptes à conférer une surpression, de préférence comprise entre 0,1 et 2 bar, de préférence encore comprise entre 0,2 et 1 bar, au circuit du gaz jusqu'aux moyens d'introduction de l'effluent sec dans le photobioréacteur ou jusqu'au contacteur gaz/liquide renfermant la solution nutritive.

Ainsi, l'effluent sec gazeux peut buller librement dans tout dispositif de carbonatation du milieu de culture ou dans tout photobioréacteur.

De manière avantageuse, l'échangeur de chaleur est relié au photobioréacteur afin de lui apporter des calories en vue d'optimiser sa température de fonctionnement et/ou au gaz sec afin de remonter sa température après sa séparation d'avec le condensat. L'installation n'est pas limitée à un certain type de photobioréacteur : l'installation peut comprendre un photobioréacteur tubulaire fermé, ou un photobioréacteur plan (sous éclairage artificiel ou naturel), ou encore un photobioréacteur ouvert, de type bassin à circulation circulaire (dénommé « raceway ») clos ou ouvert.

L'installation peut ainsi être avantageusement utilisée pour le recyclage d'effluent gazeux de cimenterie contenant du dioxyde de carbone.

L'invention sera bien comprise à la lecture de la description suivante d'exemples de réalisation, en référence aux dessins annexés dans lesquels :

### FIGURES

La figure 1 est un schéma simplifié d'un exemple d'installation pour la mise en œuvre du procédé de culture selon l'invention.
La figure 2 est un graphique montrant l'évolution de la concentration en biomasse en fonction du temps avec CO₂ ou gaz sec de cimenterie, ainsi qu'après ajout d'une fraction de condensat.

### EXEMPLES

Des expériences en phase pilote du procédé selon la présente invention ont été conduites avec deux souches différentes, inoculées dans des photobioréacteurs séparés :
- La micro-algue *Chlorella vulgaris. Chlorella vulgaris* est une algue verte unicellulaire, très riche en chlorophylle (2-3 %), mais aussi en protéines (60 % en masse), enzymes, vitamines (A, B, C et E), acides gras essentiels insaturés et minéraux (fer, calcium, magnésium, zinc, potassium, soufre, manganèse).
- La cyanobactérie *Arthrospira platensis* (Spiruline). *Arthrospira platensis,* plus connue sous le nom de « Spiruline » est une cyanobactérie filamenteuse appartenant à la famille des Cyanophycae. Ses dimensions varient de 200 à 1500 µm de long pour une épaisseur de 10 à 20 µm. En 2013, sa production mondiale était évaluée à plus de 5000 tonnes dont 300 à 500 tonnes en France. Elle est principalement utilisée comme complément alimentaire chez l'homme et/ou l'animal. En effet, elle est très riche en protéines (plus de 60% en masse), en acides gras essentiels, en vitamines, en β-carotène et en phycocyanine. Grâce à ses propriétés nutritives exceptionnelles, la Chine l'a déclarée aliment d'intérêt national et l'Organisation des Nations unies pour l'alimentation et l'agriculture (FAO) a décidé en 2014 de recommander sa production et son utilisation dans la lutte contre la malnutrition.

Au cours de cette étude, les microalgues ou cyanobactéries ont été cultivées en PhotoBioRéacteurs 1 intensifs (notés PBRs). Un photobioréacteur est un réacteur assurant la production de microorganismes photosynthétiques en suspension dans l'eau. On parle de systèmes intensifs, lorsque la plupart des paramètres sont contrôlés de façon à obtenir une productivité optimale. Ces paramètres les plus importants sont : la luminosité, la température, le pH, la concentration en nutriments, la concentration de l'espèce étudiée et la garantie des conditions axéniques de cultures (absence dans la culture, de tout organisme pouvant rentrer en compétition avec l'espèce étudiée).

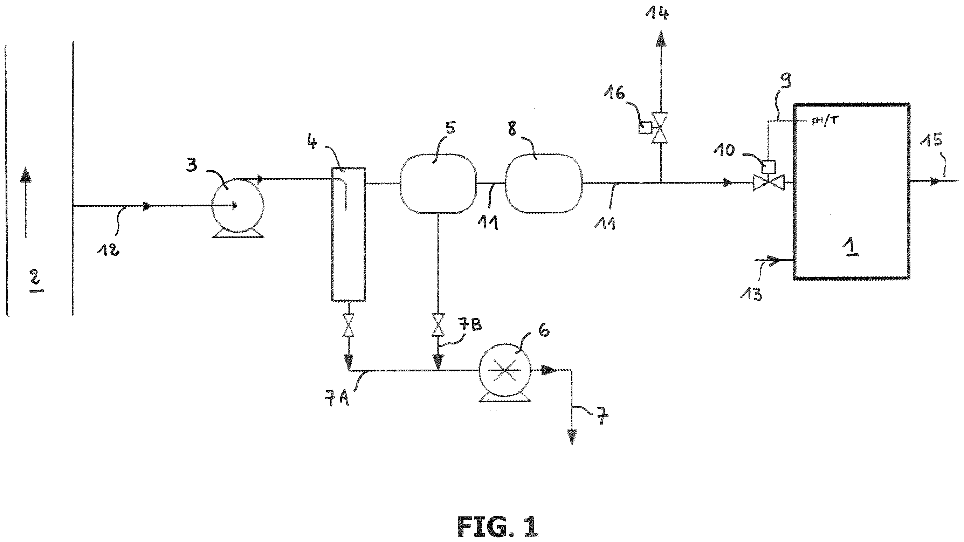

L'installation utilisée dans les exemples présentés ci-après est schématisée sur la figure 1. Cette installation a été mise en place sur un site industriel, à proximité immédiate de la cheminée 2 d'une cimenterie.

Elle comprend une pompe 3 de prélèvement direct de l'effluent gazeux 12 (dénommé aussi « fumée ») dans le conduit de la cheminée 2 de ladite cimenterie. Cette pompe 3 est reliée à un premier séparateur 4 d'une première fraction liquide 7A condensée lors de ce prélèvement. Le gaz sortant de ce séparateur 4 est ensuite refroidi à une température inférieure ou égale à 5 °C au sein du groupe froid appelé aussi refroidisseur 5 dans lequel la fraction liquide restante 7B est condensée. Ces deux fractions liquides sont collectées au moyen d'une pompe péristaltique 6 et rassemblées pour former le condensat 7 total.

Le gaz sec 11 issu du refroidisseur 5 peut être réchauffé dans un échangeur de chaleur (dénommé aussi réchauffeur 8) pour amener sa température du dit gaz sec à la température appropriée pour la culture des micro-algues ou des cyanobactéries dans le photobioréacteur 1. Ce photobioréacteur est équipé d'une sonde 9 de pH et de température. L'introduction de ce gaz sec 11 est effectuée par intermédiaire d'une vanne 10 pilotée par la sonde de pH. Le photobioréacteur peut fonctionner en mode « batch » avec récolte ponctuelle d'une partie de son contenu puis remplacement du volume prélevé par du milieu de culture frais ou en mode continu durant lequel ce dernier est alimenté en continu en solution nutritive 13 et la biomasse 15 est récoltée également en continu en vue de sa valorisation.

Enfin le surplus du gaz sec 11 est dirigé vers un évent 14 par l'intermédiaire d'une vanne 16 permettant de conserver une pression relative supérieure à environ 0,5 bar dans le circuit gazeux de l'installation.

Les conditions opératoires préférées de fonctionnement de ladite installation sont présentées ci-après.

La fumée produite par la cimenterie est filtrée dans un électrofiltre puis envoyée dans la cheminée 2 via un ventilateur d'extraction. Cet effluent gazeux qui renferme en moyenne moins de 10 mg/Nm³ de poussières en fonctionnement normal est éjecté dans l'atmosphère sous pression atmosphérique à une vitesse d'environ 20 m/s à 30 m/s et à une température comprise entre de 60°C et 90°C. Cet effluent humide contient entre 7 et 13% volumique d'humidité.

La pompe, dite de prélèvement 3, prélève en continu à un taux de 10 à 30 L/min l'effluent gazeux dans la cheminée via une tubulure en matériau synthétique ou en verre. Cette pompe permet non seulement de prélever l'effluent gazeux mais également de lui donner de l'énergie volumique (i.e. pression). La tubulure en amont et en aval de ladite pompe étant en contact avec l'atmosphère, une partie de l'humidité contenue dans l'effluent gazeux 12 prélevé se condense naturellement et est recueillie dans le séparateur 4. Afin de compléter cette première condensation « naturelle » et d'assécher au maximum l'effluent gazeux sortant du séparateur 4, celui-ci traverse le refroidisseur 5 où sa température est abaissée temporairement en dessous de 5°C (voire en dessous de 2°C). Le gaz sec 11 est ensuite réchauffé puis est envoyé en permanence à l'évent via la vanne 16 équipée d'un régulateur électronique de débit massique qui permet de maintenir une pression relative dans le circuit supérieure à 0,5 bar. Entre le réchauffeur 8 et la vanne 16 une dérivation sur la tubulure principale permet d'alimenter en gaz sec, à la demande, le photobioréacteur 1 de production de micro-algues ou de cyanobactéries.

Les condensats 7 produits, recueillis du séparateur 4 et du refroidisseur 5, sont prélevés au fur et à mesure de leur formation à l'aide de la pompe péristaltique 6 dédiée qui permet également de maintenir la pression dans la ligne de prélèvement. Ces condensats incluent non seulement l'humidité de la fumée produite par la cimenterie mais également la grande majorité des poussières, des éléments métalliques et des imbrûlés. Ce système de traitement de l'effluent gazeux brut de la cimenterie permet donc de purifier celui-ci de manière continue.

Dans les essais réalisés, le gaz sec 11 issu de la cimenterie présentait la composition moyenne suivante : entre 5% et 20% de CO₂, entre 5% et 20% de O₂ et entre 60% et 90% de N₂ volumiques. Il renfermait par ailleurs des imbrûlés en très faibles proportions, des NOx entre 300 et 700 mg/Nm³ et du CO entre 100 et 300 mg/Nm³.

### Exemple 1 :

Paramètres de la fumée de cimenterie : P = Pₐₜₘ ; T = 80°C ; Poussières totales : 10 mg/Nm³ ; taux d'humidité : 9%
Prélèvement de fumée : 20 L/min
Pression relative en aval de la pompe de prélèvement : +0,6 bar
Température de la fumée dans le refroidisseur : +3°C
Composition volumique principale du gaz sec obtenu : CO₂ = 13% ; O₂ = 13% ; N₂ = 74% volumiques ; NOx = 600 mg/Nm³ ; CO = 200 mg/Nm³

Les résultats obtenus ont permis de constater que le procédé appliqué avait permis de réduire au moins :
> 90% de la fraction humide de la fumée de cimenterie
> 95% des poussières totales en masse (incluant les poussières de cru et les métaux y compris les métaux lourds)
> 99% de HCl + NH₃ + HF
> 60% des imbrûlés
> 15% des NOx

Les analyses des eaux de condensation (condensats) montrent que l'on y retrouve des constituants du cru et du clinker (de la cimenterie) tels que : calcium, potassium, sodium, soufre, silicium, magnésium, aluminium, fer (appelés éléments « majeurs » : calcium entre 40 et 80 mg/L, potassium de 6 à 10 mg/L, tandis que les éléments aluminium, magnésium, sodium, soufre et silice sont presque tous dans une gamme de 1 à 7 mg/L).

On retrouve dans les eaux de condensation des ions nitrites (de 50 à 100 mg/L) et des ions nitrates (de à 10 mg/L) ainsi que des traces d'imbrûlés.

On y retrouve également des éléments métalliques en très faibles proportions (appelés éléments « mineurs ») : les concentrations en arsenic (<20 µg/L), béryllium (<5 µg/L), cadmium (<4 µg/L), cobalt (<10 µg/L), molybdène (<10 µg/L), antimoine (<6 µg/L) et tellure (<10 µg/L) sont en dessous de la limite de détection de l'ICP (indiquée pour chaque élément entre parenthèses). Les concentrations en chrome (<8 µg/L), manganèse (<10 µg/L), nickel (<5 µg/L), sélénium (<10 µg/L), étain (<10 µg/L), thallium (<15 µg/L) et vanadium (<10 µg/L), sont parfois proches de cette limite ou en dessous. Enfin, on remarque que pour les éléments qui sont en général au-dessus du seuil de détection, deux groupes se démarquent nettement :
- Ceux dont les valeurs conservent le même ordre de grandeur : baryum, chrome, nickel, sélénium, étain, strontium et vanadium avec respectivement des moyennes de concentrations à 31,3 µg/L, 14,3 µg/L, 19,6 µg/L, 20,3 µg/L, 5 µg/L, 246 µg/L et 18,2 µg/L.
- Ceux dont les valeurs fluctuent sensiblement d'un essai à l'autre : mercure (14 à 245 µg/L), cuivre (514 à 3956 µg/L), fer (21 à 4741 µg/L) plomb (18 à 143 µg/L), thallium (<15 à 182 µg/L) et zinc (761 à 6778 µg/L).

Tous les éléments ci-dessus, détectés dans les eaux de condensation sont donc éliminés de la fraction gazeuse dans le gaz sec 11 entrant dans le photobioréacteur 1.

### Exemple 2 :

Néanmoins, afin d'évaluer la robustesse du procédé, c'est-à-dire, d'évaluer la capacité des cultures à tolérer d'éventuels passages ou ajouts de condensat dans le milieu de culture, afin également de gérer au mieux les condensats formés lors du prélèvement du gaz de cimenterie, ont été réalisées des expériences dans lesquelles le taux de condensat dans le milieu de culture alimentant en continu le photobioréacteur test était augmenté à 2,5%, puis à 5% (volumique). Soulignons qu'un ajout de 0,2 % de condensat dans le milieu de culture correspond à une mise en contact des cultures avec le gaz humide dans sa totalité (effluent produit par la cimenterie).

Les micro-algues ont été cultivées dans un photobioréacteur tubulaire vertical de 5 cm de diamètre interne et de volume 6,4 L.

Les conditions de fonctionnement sont regroupées dans le tableau 1 suivant :

**Tableau 1 : Tableau récapitulatif des conditions de culture pour les expériences d'évaluation de la robustesse des cultures avec les PBRs tubulaires**

| Souche | *Arthrospira platensis* |
|---|---|
| Débit de bullage d'air | 300 ± 20 mL/min d'air |
| pH | 9,50 ± 0,01 |
| Température | 35 ± 2°C |
| Luminosité | 4 néons de 21 Watts en formation interne ou externe |
| Injection de CO₂ (13 %) | 45 ± 2 mL/min de CO₂ pur |
| | 300 ± 20 mL/min de gaz de cimenterie |
| Débit de milieu de culture | 1,5 mL/min |
| Taux de dilution | 0,014 h⁻¹ |
| Milieu de culture | Cf. Tableau 2 |

Ce système a fonctionné en mode « continu » avec ajout de 2,2 mL/min de milieu de culture frais permettant également une récolte en continu de la biomasse 15 produite. En lumière artificielle, pour une souche donnée, ce système consomme en moyenne 1,7 L/h de gaz sec (qui correspond à 0,11 mL de condensat pour une fumée renfermant au départ 9% d'humidité).

Ce condensat, riche en oligo-éléments ainsi qu'en ions nitrites et ions nitrates (source d'azote), peut être ajouté au milieu de culture qui alimente en continu le photobioréacteur tubulaire.

Deux photobioréacteurs tubulaires mettaient en œuvre des cultures similaires en parallèle :
- Un premier PBR tubulaire servant de référence, alimenté par du CO₂ pur et un milieu de culture sans ajout de condensat.
- Un second PBR tubulaire constituant la culture « test », alimenté par du gaz sec de cimenterie et, en mode continu, un milieu de culture renfermant des concentrations volumiques croissantes en condensat (0,2%, 2,5% et 5%).

Le milieu de culture est détaillé dans le tableau 2 ci-dessous.

**Tableau 2 : Composition du milieu de culture pour la souche Arthrospira platensis**

| Eléments majeurs | Elément | Masse en g/L (ou kg/m³) | Elément | Masse en g/L (ou kg/m³) |
|---|---|---|---|---|
| | NaCl | 1 | K₂SO₄ | 0,4 |
| | CaCl₂,2H₂O | 0,012 | MgSO₄,7H₂O | 0,032 |
| | NaNO₃ | 1 | NaHCO₃ | 4,2 |
| | FeSO₄,7H₂O | 0,005 | Na₂CO₃ | 3,04 |
| | Na₂EDTA | 0,04 | K₂HPO₄ | 0,2 |
| Oliqoéléments 1 mL/L de milieu de culture | Elément | Masse en g/L (ou kg/m³) | Elément | Masse en g/L (ou kg/m³) |
| | MnCl₂,4H₂O | 0,23 | CUSO₄,5H₂O | 0,03 |
| | ZnSO₄,7H₂O | 0,11 | | |

La biomasse produite dans chacun des photobioréacteurs a été récoltée, filtrée (sur tamis de 38 µm puis sur filtre GF/F de 0,7 µm), séchée, puis analysée. Le filtrat a également été analysé pour détecter les éléments non absorbés par la biomasse.

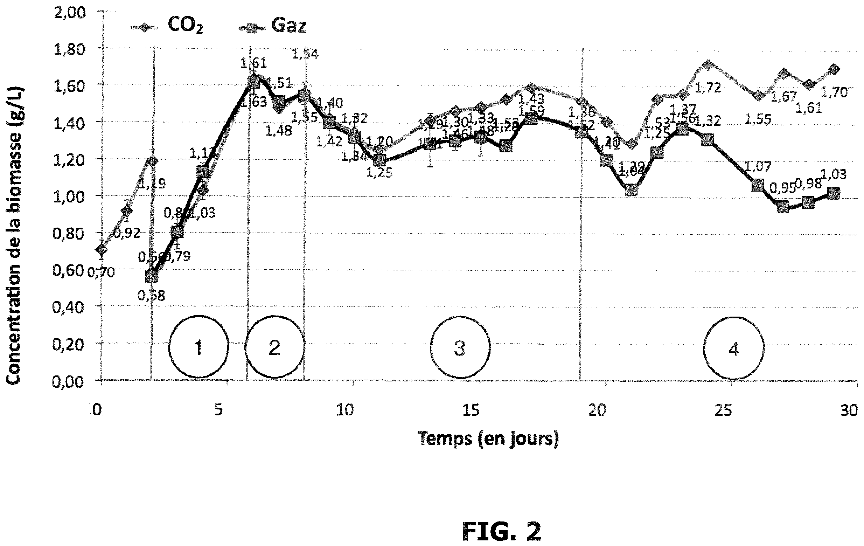

Le graphe de la figure 2 présente l'évolution de la concentration en biomasse dans les deux PBRs tubulaires en fonction du temps. Sur ce graphe, la première ligne verticale schématise le lancement de la phase dite « batch » et la seconde, le passage de la culture du mode batch au mode continu. Plus particulièrement, au temps 0 a été injecté 1,1 L d'inoculum dans le photobioréacteur de référence. Après une montée en concentration, un transfert d'une moitié de la biomasse du PBR de référence vers le PBR test a été effectué à t= 2 jours, démarrage de la phase dite « batch ». Après une nouvelle montée en concentration dans les deux PBRs, le mode continu est lancé dans ces dernières à t = 6 jours. Les autres lignes verticales indiquent le passage à une phase en mode continu avec ajout de condensat dans le milieu de culture (pour le PBR test) :
1 : Phase « batch »
2 : Phase continue sans ajout de condensat
3 : Phase avec un ajout de 2,5% de condensat dans le PBR test
4 : Phase avec un ajout de 5 % de condensat dans le PBR test

On remarque que l'évolution des concentrations des deux PBRs se déroule de manière identique de la phase 1 jusqu'au premier tiers de la phase 3. À partir du 11^{e} jour, la concentration du PBR référence, est toujours supérieure à celle du PBR test. Néanmoins, les concentrations évoluent de manière similaire jusqu'au premier tiers de la phase 4. Dès lors, la concentration du PBR référence reste stable autour de 1,68 g/L, alors que celle du test passe de 1,32 g/L, à 0,99 g/L en l'espace de trois jours. Les débits de dilution ne sont pas responsables de cette évolution différente des concentrations. Au contraire, la moyenne du débit de dilution pour le témoin est de 1,51 mL/min, alors que celle du PBR test est de 1,38 mL/min. Ainsi, même avec un débit de dilution moins important que pour le PBR de référence, à partir du premier tiers de la phase 3 (2,5% de condensat dans le milieu de culture), le taux de croissance du PBR test est moins important que celui du PBR de référence. Ce contraste est clairement dû à l'ajout de 2,5 % de condensat (ajout fait dans le milieu de culture test, mais également directement dans le PBR test, le 8^{e} jour).

Par ailleurs, des analyses ont notamment été réalisées pour déterminer la concentration des différents métaux présents dans la souche *Arthrospira* platensis produite avec 0,2 %, 2,5 % et 5 % de condensat (il en a été de même avec la souche *Chlorella vulgaris* séchée et produite avec 0,1 % et 10% de condensat ajouté au milieu de culture).

Les résultats relatifs à la Spiruline sont regroupés dans le tableau 3 ci-après.

**Tableau 3 : Analyse des métaux présents dans la souche Spiruline**

| Essais | | 1 | | 2 | | 3 | | 4 | | 5 | |
|---|---|---|---|---|---|---|---|---|---|---|---|
| PRB/ Eléments | Réglementation (mg/kg) | Réf. | Test 0,2% | Réf. | Test 0,2% | Réf. | Test 2,5% | Réf. | Test 2,5% | Réf. | Test 5% |
| Arsenic minéral | 3 | 0,52 | 0,36 | 0,67 | 0,54 | <1,00 | <1,00 | <1,00 | <1,00 | <1,00 | <1,00 |
| Cadmiu m | 0,5 | 0,06 | 0,05 | 0,15 | 0,19 | <0,20 | <0,20 | <0,20 | <0,20 | <0,20 | <0,20 |
| Mercure | 0,1 | 0,05 | 0,02 | 0,01 | 0,01 | 0 | 0,08 | 0,04 | 0,12 | 0,03 | 0,19 |
| Plomb | 5 | 1,42 | 1,62 | 2,96 | 2,63 | 0,76 | 0,77 | 7,33 | 3,46 | 0,91 | 2,36 |
| Etain | 5 | 2,12 | 1,83 | 3,55 | 3,77 | 1,08 | 0,52 | 0,85 | 0,99 | 0,73 | 0,84 |

Les différents essais réalisés avec une biomasse de Spiruline produite avec une solution nutritive renfermant différentes teneurs en condensat, n'a pas révélé, pour des teneurs inférieures à 2,5 % (notamment pour une teneur de 0,2 %) d'impact sur la croissance de ces cyanobactéries (résultats non présentés), ni d'accumulation dans celles-ci des principaux éléments métalliques (dont notamment le plomb) à prendre en compte dans l'alimentation humaine (selon avis du CSHPF Saisine AFSSA n° 2007-SA-0007). En revanche, pour des teneurs de 2,5 % et plus, l'impact sur la croissance des cyanobactéries est significatif (voir figure 2) ainsi que l'accumulation de certains métaux dans les cellules comme le mercure (voir tableau 3).

Ces essais montrent d'une part la robustesse du procédé selon l'invention, et d'autre part que si le milieu de culture ajouté en continu contient jusqu'à 0,2% de condensat 7, les différentes souches testées se développent normalement et la biomasse ainsi produite respecte les normes alimentaires européennes. La spiruline ainsi produite pourrait donc être utilisée comme complément alimentaire, à l'état pur ou après extraction de ses principes actifs.

### Exemple 3 :

Des tests effectués en laboratoire sur la micro-algue *Nannochloropsis oculata* en présence de différents ajouts de condensats (1 %, 5 %, 10 %, 15 %) provenant de la même cimenterie que celle des exemples 1 et 2 ci-dessus ont montré que cette micro-algue présente une résistance remarquable aux éléments contenus dans les condensats.

Plus particulièrement la micro-algue *Nannochloropsis oculata* possède une capacité à accumuler des lipides au sein des cellules (avec des teneurs supérieures à 50 % - 70 % en masse) lorsque son "alimentation" en milieu nutritif est carencée en élément azote. Cette micro-algue présente donc un intérêt non négligeable en termes d'accumulation de lipides de type "acides gras à doubles liaisons multiples" (polyinsaturés) et donc d'applications dans les domaines de l'alimentation et des biocarburants.

Les mêmes tests effectués en laboratoire ont été réalisés sur les microalgues *Chlorella vulgaris* et *Arthrospira platensis* (Spiruline). La micro-algue *Chlorella vulgaris* a montré également une résistance remarquable aux mêmes ajouts de condensats (1 %, 5 %, 10 %, 15 %) ainsi qu'à un ajout de 30 %. La cyanobactérie *Arthrospira platensis* n'est quant à elle pas affecté par un ajout de 1 % de condensats à son milieu de culture (confirmation des résultats obtenus en cimenterie avec un ajout de 0,2 % - voir exemple 2). En revanche, son développement est affecté avec un ajout de 10 % de condensats à son milieu de culture (précédemment, même constat avec un ajout de 2,5 % et de 5 % de condensats à son milieu de culture).

Les conclusions principales de l'ensemble de l'étude à retenir sont :
- qu'au contact de l'effluent gazeux de cimenterie utilisé comme source de CO₂ (ordre de grandeur d'ajout du condensât : 0,1 % - 0,2 %), les trois souches mentionnées dans la présente demande de brevet se développent normalement.
- que les procédés de culture en recyclant l'effluent gazeux sont robustes, c'est-à-dire qu'en multipliant par 5 ces taux, les micro-algues continuent à se développer normalement et que leur taux en métaux intracellulaires respecte les normes alimentaires.
- qu'à des taux supérieurs d'ajout de condensats (> 1 %), les souches *Chlorella vulgaris* et *Nannochloropsis oculata* continuent à présenter une bonne résistance aux condensats.

## Revendications

1. Procédé de culture de micro-algues et/ou de cyanobactéries dans un photobioréacteur (1) renfermant un milieu nutritif incluant une source carbonée, la source carbonée est un effluent de gaz industriel contenant du dioxyde de carbone, et le procédé comprend les étapes successives suivantes :
a) - Le captage de l'effluent gazeux industriel humide contenant du dioxyde de carbone,
b) - Le séchage/purification dudit effluent gazeux par refroidissement, provoquant la condensation de la fraction humide et l'obtention d'un effluent gazeux sec,
c) - La séparation du condensat de l'effluent gazeux sec contenant du dioxyde de carbone,
d) - le suivi du pH du milieu de culture dans le photobioréacteur renfermant une ou plusieurs souches de micro-algues et/ou cyanobactéries, avec détermination d'une gamme de pH optimale pour la croissance desdites souches,
e1) - L'enrichissement en dioxyde de carbone (carbonatation) d'une solution nutritive des micro-algues et/ou cyanobactéries au moyen dudit effluent sec/purifié, suivi, en fonction du pH du milieu de culture, de l'injection de ladite solution nutritive enrichie en dioxyde de carbone dans le photobioréacteur,
ou e2) - l'injection directe de l'effluent sec contenant le dioxyde de carbone dans le photobioréacteur,
f) - La récolte de la biomasse produite en vue de sa valorisation,
**caractérisé en ce que** l'effluent gazeux est un effluent issu de l'industrie cimentière, plus particulièrement capté dans le conduit de la cheminée (2) d'une cimenterie, avant son rejet dans l'atmosphère, **en ce que** le refroidissement de l'effluent est effectué à une température inférieure ou égale à 5°C, et **en ce que** les micro-algues ou cynanobactéries sont choisies parmi les souches suivantes : *Arthrospira platensis* (spiruline), *Chlorella vulgaris* ou *Nannochloropsis oculata.*

2. Procédé selon la revendication 1, **caractérisé en ce que** ledit procédé est réalisé en continu.

3. Procédé selon l'une quelconque des revendications précédentes, **caractérisé en ce qu'**au moins une fraction du condensat est introduite dans le photobioréacteur.

4. Procédé selon l'une quelconque des revendications précédentes, **caractérisé en ce que** le refroidissement de l'effluent est effectué à une température inférieure ou égale à 2 °C.

5. Procédé selon l'une quelconque des revendications précédentes, **caractérisé en ce qu'**au moins une partie des calories récupérées lors du refroidissement de l'effluent brut sont dirigées vers le photobioréacteur et/ou vers le gaz sec.

6. Procédé selon l'une quelconque des revendications précédentes, **caractérisé en ce que** les différentes étapes comprenant le séchage/purification de l'effluent par refroidissement, la séparation de l'effluent sec et du condensat, l'enrichissement en dioxyde de carbone du milieu de culture des micro-algues et l'injection dudit milieu de culture enrichi en dioxyde de carbone dans un photobioréacteur sont réalisées sous une pression relative comprise entre 0,1 et 2 bar, de préférence sous une pression relative comprise entre 0,2 et 1 bar.

## Patentansprüche

1. Verfahren zur Kultivierung von Mikroalgen und/oder Cyanobakterien in einem Photobioreaktor (1), der ein Nährmedium einschließt, das eine Kohlenstoffquelle umfasst, wobei die Kohlenstoffquelle Industrieabgase sind, die Kohlendioxid enthalten, wobei das Verfahren die folgenden aufeinanderfolgenden Schritte umfasst:
a) - das Auffangen der nassen kohlendioxidhaltigen Industrieabgase,
b) - die Trocknung/Reinigung der Abgase durch Kühlung, wodurch die Kondensation des Nassanteils und der Erhalt trockener Abgase bewirkt wird,
c) - die Trennung des Kondensats von den trockenen kohlendioxidhaltigen Abgasen,
d) - die Verfolgung des pH-Werts des Kulturmediums in dem Photobioreaktor, der einen oder mehrere Stämme von Mikroalgen und/oder Cyanobakterien einschließt, mit Bestimmung eines für das Wachstum der Stämme optimalen pH-Werts,
e1) - die Anreicherung einer Nährlösung der Mikroalgen und/oder Cyanobakterien mit Kohlendioxid (Karbonisierung) mittels der trockenen/gereinigten Abgase in Abhängigkeit des pH-Werts der Kultur, gefolgt von dem Einpressen der mit Kohlendioxid angereicherten Nährlösung in den Photobioreaktor,
oder e2) - das direkte Einpressen der trockenen kohlendiodixhaltigen Abgase in den Photobioreaktor,
f) - die Sammlung der hergestellten Biomasse im Hinblick auf ihre Verwertung, **dadurch gekennzeichnet, dass** die Abgase Abgase sind, die von der Zementindustrie stammen, die insbesondere im Kanal des Schornsteins (2) einer Zementfabrik aufgefangen werden, vor ihrer Freisetzung in die Atmosphäre, dadurch, dass die Abkühlung der Abgase bei einer Temperatur von niedriger oder gleich 5 °C durchgeführt wird, und dadurch, dass die Mikroalgen oder Cyanobakterien aus den folgenden Stämmen ausgewählt werden: *Arthrospira platensis* (Spirulina), *Chlorella vulgaris* oder *Nannochloropsis oculata.*

2. Verfahren nach Anspruch 1, **dadurch gekennzeichnet, dass** das Verfahren ununterbrochen ausgeführt wird.

3. Verfahren nach einem der vorhergehenden Ansprüche, **dadurch gekennzeichnet, dass** mindestens ein Anteil des Kondensats in den Photobioreaktor eingeleitet wird.

4. Verfahren nach einem der vorhergehenden Ansprüche, **dadurch gekennzeichnet, dass** die Abkühlung der Abgase bei einer Temperatur von niedriger oder gleich 2 °C durchgeführt wird.

5. Verfahren nach einem der vorhergehenden Ansprüche, **dadurch gekennzeichnet, dass** zumindest ein Anteil der bei der Abkühlung der Rohabgase wiedergewonnenen Kalorien in Richtung des Photobioreaktors und/oder in Richtung des trockenen Abgases geleitet werden.

6. Verfahren nach einem der vorhergehenden Ansprüche, **dadurch gekennzeichnet, dass** die verschiedenen Schritte die Trocknung/Reinigung des Abgases durch Kühlung, die Trennung des trockenen Abgases und des Kondensats, die Anreicherung des Kulturmediums der Mikroalgen mit Kohlendioxid und das Einpressen des mit Kohlendioxid angereicherten Kulturmediums in einen Photobioreaktor unter einem Überdruck von zwischen 0,1 und 2 Bar, vorzugsweise unter einem Überdruck von zwischen 0,2 und 1 Bar, ausgeführt werden.

## Claims

1. A process for microalgae and/or cyanobacteria cultivation in a photobioreactor (1) containing a nutrient medium including a carbon source, the carbon source being an industrial gaseous effluent containing carbon dioxide, and the process comprising the following successive steps:
a) - Capturing the wet industrial gaseous effluent containing carbon dioxide,
b) - Drying/purifying said gaseous effluent by cooling, causing the condensation of the wet fraction and obtaining a dry gaseous effluent,
c) - Separating the condensate of the dry gaseous effluent containing carbon dioxide,
d) - Following the pH of the culture medium in the photobioreactor containing one or more strains of microalgae and/or cyanobacteria, with the determination of an optimal pH range for the growth of said strains,
e1) - Enriching with carbon dioxide (carbonation) a nutrient solution of microalgae and/or cyanobacteria by means of said dry/purified effluent, as a function of the pH of the culture medium, of the injection of said nutrient solution enriched with carbon dioxide into the photobioreactor,
or e2) - Direct injection of the dry effluent containing carbon dioxide into the photobioreactor,
f) - Collection of the biomass produced in view of the exploitation thereof, **characterised in that** the gaseous effluent is an effluent that comes from the cement industry, more particularly captured in the conduit of the stack (2) of a cement works, being discharged into the atmosphere, and **in that** the cooling of the effluent is performed at a temperature less than or equal to 5°C, and **in that** the microalgae or cyanobacteria are chosen from the following strains: *Arthrospira platensis* (spirulina), *Chlorella vulgaris* or *Nannochloropsis oculata.*

2. The process according to claim 1, **characterised in that** said process is carried out continuously.

3. The process according to any one of the preceding claims, **characterised in that** at least one fraction of the condensate is introduced into the photobioreactor.

4. The process according to any one of the preceding claims, **characterised in that** the cooling of the effluent is performed at a temperature less than or equal to 2°C.

5. The process according to any one of the preceding claims, **characterised in that** at least a part of the calories recovered during the cooling of the raw effluent are directed towards the photobioreactor and/or towards the dry gas.

6. The process according to any one of the preceding claims, **characterised in that** the different steps comprising the drying/purification of the effluent by cooling, the separation of the dry effluent and of the condensate, the enriching in carbon dioxide of the culture medium of the microalgae and the injection of said culture medium enriched with carbon dioxide into a photobioreactor are performed under relative pressure comprised between 0.1 and 2 bar, preferably under relative pressure comprised between 0.2 and 1 bar.
